# FASCICULE DE BREVET EUROPEEN

(11) **EP 2 170 412 B1**
(45) Date de publication et mention de la délivrance du brevet: **25.03.2015**
(21) Numéro de dépôt: 08826730.7
(22) Date de dépôt: 25.07.2008
(51) Int. Cl.: A61L 15/28, A61L 15/64, C08B 15/04, D06M 11/07, D06M 11/64

(54) **PROCEDE DE PREPARATION DE COMPRESSE DE CELLULOSE OXYDEE**
VERFAHREN ZUR HERSTELLUNG EINER KOMPRESSE AUS OXIDIERTER ZELLULOSE
METHOD FOR PREPARING AN OXIDISED CELLULOSE COMPRESS

(30) Priorité: 26.07.2007 FR 0705477
(43) Date de publication de la demande: 07.04.2010
(73) Titulaire: Symatese, 69630 Chaponost (FR)
(72) Inventeur: BERTHOLON, Cécile, F-69360 Ternay (FR); DAVID, Céline, F-38190 Villard-Bonnot (FR); HERBAGE, Benjamin, F-69350 La Mulatiere (FR)
(74) Mandataire: Denjean, Eric
(86) Numéro de dépôt international: PCT/FR2008/051413
(87) Numéro de publication internationale: WO 2009/016325

(56) Documents cités:
- EP-A- 1 245 722
- EP-A- 1 400 624
- WO-A-2007/104267
- US-A- 983 073
- US-A- 2 496 797
- US-A1- 2002 072 598

## Description

### DOMAINE DE L'INVENTION

L'invention concerne un nouveau procédé d'oxydation des compresses de cellulose.

Après oxydation, les compresses ainsi obtenues présentent un caractère hémostatique et résorbable, adapté à une application en tant que compresses implantables.

Plus précisément, le procédé proposé dans le cadre de l'invention est mis en oeuvre directement sur les compresses de cellulose. Il repose sur l'oxydation de la fonction alcool primaire des résidus glucose de la cellulose par un sel d'oxoammonium en présence d'un système oxydant secondaire. Ce procédé, réalisé en solution aqueuse, est aisément mis en oeuvre à l'échelle industrielle.

### ETAT DE L'ART ANTERIEUR

Les compresses hémostatiques chirurgicales ou compresses doivent être hémostatiques, résorbables et facilement manipulables pour les chirurgiens. Ces propriétés peuvent être obtenues grâce à des textiles à base de cellulose oxydée.

Pour rappel, la cellulose (C₆H₁₀O₅)ₙ est un homopolymère appartenant à la classe des polysaccharides. Elle est formée d'un enchaînement linéaire de molécules de glucose par des liaisons glycosidiques β-1,4.

Il est connu de l'art antérieur que les propriétés hémostatique et résorbable de la cellulose oxydée sont obtenues par oxydation sélective du groupe alcool porté par le carbone 6 de l'unité d'anhydroglucose, en acide carboxylique. Toute la difficulté repose donc dans l'oxydation sélective d'un alcool primaire, en présence de fonctions alcool secondaire.

Les compresses sont généralement fournies avec des fonctions acide carboxylique protonées ou complexées à l'aide de l'ion calcium (Ca²⁺), comme décrit par exemple dans les documents GB 1 593 513 et US 5 484 913.

Actuellement, de telles compresses sont obtenues en soumettant le textile de cellulose à l'action du NO₂ en présence de solvants. Plus précisément, les compresses sont plongées dans un bain de solvant avec barbotage de NO₂ gazeux, tel que décrit par exemple dans le document EP 0 492 990.

EP1400624, US98307 et US2496797 décrivent l'utilisation des compresses biorésorbables et hémostatiques. Ces compresses sont composées de cellulose oxydée. EP1245722 décrit un procédé de préparation d'une pulpe de cellulose oxydée comprenant l'oxydation de cette pulpe à l'aide d'un hypohalite, en présence d'un sel d'oxoammonium. Une compresse est formée de la cellulose oxydée.

Toutefois, de tels procédés ne sont pas satisfaisants car ils requièrent des matières premières coûteuses, dangereuses et difficiles à recycler, ainsi qu'une installation exigeante.

Il existe donc un besoin de développer de nouveaux procédés, applicables industriellement et moins contraignants, permettant d'obtenir des compresses de cellulose oxydée, satisfaisantes en termes de degré d'oxydation et de résistance mécanique.

De manière surprenante, le Demandeur a montré qu'une autre technique d'oxydation sélective d'alcool primaire pouvait être mise en oeuvre avec succès, directement sur des compresses à base de cellulose.

### EXPOSE DE L'INVENTION

Ainsi, l'invention concerne un nouveau procédé de préparation de compresses en cellulose oxydée.

Pour l'essentiel, un tel procédé comprend l'oxydation de la compresse à base de cellulose à l'aide d'un hypohalite, en présence d'un sel d'oxoammonium.

Ce procédé est donc mis en oeuvre directement sur une compresse à base de cellulose comme matériel de départ, c'est-à-dire un textile déjà mis en forme.

On entend par « à base de cellulose », le fait que la compresse contient des fibres qui chimiquement s'apparentent à la cellulose de par leur nature (homopolymères de glucose avec des liaisons glycosidiques β-1,4). Toutefois, outre la cellulose, il peut par exemple s'agir de viscose qui correspond à un fil obtenu à partir de cellulose régénérée.

Dans une étape antérieure au procédé selon l'invention, les fibres ou fils sont assemblés sous forme de textile, qui peut être aussi bien un tissé ou un tricot qu'un non-tissé.

Dans un mode de réalisation privilégié, le fil de viscose est mis sous forme de textile à l'aide du tricotage. Différentes mailles peuvent être envisagées, notamment le jersey (classiquement tricoté avec un fil de 220 dtex à 42 filaments) ou le crochet (avantageusement tricoté avec du fil 110 dtex à 40 filaments). Dans le cadre de l'invention, la maille crochet est préférée.

Typiquement et pour l'application compresse, le textile présente un grammage de l'ordre de 50 à 100 g/m².

Egalement en amont du procédé selon l'invention, le textile ainsi obtenu est avantageusement soumis à une étape de désensimage permettant l'élimination des corps gras utilisés dans la filature. Ces corps gras sont essentiellement constitués d'huiles d'ensimage et de colle acrylate enduits autour des filaments.

Différents protocoles de désensimage sont connus de l'art antérieur et peuvent être mis en oeuvre sur le textile, notamment :
- lavage avec de la lessive et de l'eau chaude (60°C) ;
- lavage avec du cyclohexane ou d'autres solvants des corps gras ;
- lavage avec des détergents ioniques ou non, par exemple ceux de la société Thorquest.

La première étape du procédé selon l'invention est donc celle permettant l'oxydation sélective des groupements alcool primaire situés en position 6 des unités d'anhydroglucose présentes dans la compresse.

Cette oxydation est réalisée grâce à l'incubation de la compresse à base de cellulose dans un milieu comprenant un hypohalite et un sel d'oxoammonium.

Cette réaction d'oxydation présente, comme premier avantage, de se dérouler en milieu aqueux et donc sans solvant, contrairement aux méthodes de l'art antérieur.

Les sels d'oxoammonium sont des oxydants hydrosolubles capables d'oxyder sélectivement les alcools primaires, lorsque la réaction se déroule dans des conditions appropriées de pH et de température. Les sels d'oxoammonium privilégiés selon l'invention sont les sels di-tert-alkylés, en particulier les sels de 2,2,6,6-tétraméthylpipéridine-1-oxyde appelés communément TEMPO.

Dans une telle réaction, ils jouent le rôle de catalyseur et sont avantageusement présents dans le milieu réactionnel à hauteur de 0,05 mole par mole d'unités anhydroglucose dans l'échantillon de cellulose traité (équivalent).

Les sels d'oxoammonium doivent donc être régénérés *in situ* par un système oxydant secondaire ou auxiliaire, en l'occurrence en présence d'un hypohalite, préférentiellement l'hypochlorite de sodium (NaOCl), tel que l'eau de javel à 15% en chlore actif qui est un réactif courant et peu coûteux.

En fait, il s'agit d'un système oxydant puisqu'il comprend, outre l'hypohalite, un sel (avantageusement un bromure) capable d'assurer le transfert de l'oxygène entre l'ion oxoammonium et l'hypohalite. Cet « intermédiaire » est avantageusement présent dans le milieu réactionnel à hauteur d'au moins 0,5 mole par mole d'unités anhydroglucose dans l'échantillon de cellulose traité (équivalent).

Un système oxydant privilégié selon l'invention est le système NaOCl/NaBr (bromure de sodium). L'ion hypobromite étant plus réactif que l'ion hypochlorite, la régénération rapide des ions oxoammonium a lieu selon le schéma suivant :

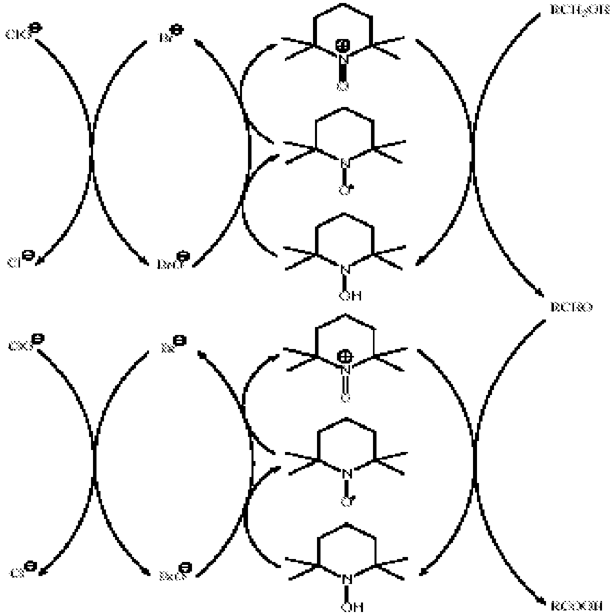

Il est admis que l'utilisation de ces réactifs pour l'oxydation des alcools primaires avait déjà été décrite, y compris dans le contexte des hydrates de carbone.

Toutefois, rien n'indiquait dans l'art antérieur que cette réaction d'oxydation pouvait être réalisée avec succès directement sur un textile à base de cellulose et donnerait lieu à une compresse présentant un taux d'oxydation supérieur ou égal à 10% (valeur correspondant à des compresses biorésorbables), voire 12% (valeur correspondant à des compresses biorésorbables et hémostatiques) et même au-delà de 14%, compatible avec l'application visée, l'oxydation étant de plus sélective des groupements alcool portés par le carbone 6 des unités anhydroglucose de la cellulose en présence.

En outre, le Demandeur a déterminé les conditions de traitement d'une telle compresse, de manière à ce qu'elle ne perde pas ses caractéristiques mécaniques, également primordiales dans l'application visée.

De manière avantageuse, le milieu réactionnel pour l'oxydation est formulé dans de l'eau déminéralisée, de manière à ne pas perturber la force ionique et le pH.

Comme décrit dans l'art antérieur, la réaction d'oxydation se déroule avantageusement à basse température, par exemple à 4°C. Ces conditions de température ont été décrites comme augmentant la sélectivité de la réactivité du groupement alcool primaire, vis-à-vis des alcools secondaires. Elles permettent en outre de limiter la perte d'intégrité mécanique du textile.

De même, la réaction se déroule avantageusement à pH basique, avantageusement compris entre 7 et 12.

Il a été montré par le Demandeur qu'un ajout trop brusque de l'hypohalite pouvait affecter la tenue de la compresse. C'est pourquoi dans un mode de réalisation privilégié, l'hypohalite est ajouté graduellement dans le milieu réactionnel, au fur et à mesure de sa consommation.

Au début de la réaction et de manière privilégiée, l'hypohalite est ajouté au milieu réactionnel, de manière à ce que le pH initial soit compris entre environ 10 et 12.

La consommation de l'hypohalite se traduit par une diminution du pH. Celui-ci est surveillé et lorsqu'il atteint les valeurs inférieures permises pour la sélectivité de la réaction, à savoir un pH compris entre 7 et 9, avantageusement égal à 8, de l'hypohalite est ajouté dans le milieu réactionnel de manière à remonter le pH.

L'ajout d'hypohalite est stoppé lorsqu'un nombre de moles d'hypohalite par mole d'unités anhydroglucose dans l'échantillon de cellulose traité (équivalents). compris entre 5 et 7, avantageusement de l'ordre de 7, a été consommé.

En pratique, l'ajout de l'hypohalite peut se faire en 8 fois sur une durée de 3 à 4 heures.

Lorsque l'ajout d'hypohalite est terminé, la réaction d'oxydation se poursuit jusqu'à l'atteinte d'un pH palier compris entre 5 et 8. Ce pH palier est en général atteint après 24 heures de réaction.

Un moyen radical pour stopper la réaction d'oxydation est d'ajouter au milieu réactionnel un alcool primaire, tel que du méthanol ou de l'éthanol, qui va réagir préférentiellement avec le sel d'oxoammonium.

En pratique et en présence de Tempo, la fin de la réaction se traduit par un milieu réactionnel qui, du jaune initial, devient blanc.

Comme déjà dit, une compresse de cellulose oxydée prête à l'emploi doit présenter soit des fonctions acide carboxylique protonées, soit complexées avec des ions calcium (Ca²⁺), pour développer les propriétés hémostatiques recherchées.

Ainsi, plusieurs options s'offrent pour la suite du procédé :

Dans le cas où il est souhaité une cellulose oxydée présentant des fonctions acides carboxylique protonées, le procédé selon l'invention implique une étape supplémentaire de protonation.

De manière classique, cette étape est réalisée par incubation de la compresse dans un milieu de protonation.

Ceci est avantageusement réalisé par incubation de la compresse dans de l'acide chlorhydrique (HCl), avantageusement dans un ou plusieurs bains de HCl 1 mol/l (N), pendant plusieurs heures. Ainsi, au moins deux bains d'une durée totale d'au moins 2 h30 assurent la protonation de l'ensemble des fonctions oxydées.

En pratique et pour éviter des manipulations de la compresse traitée, le milieu d'oxydation peut être éliminé par vidange.

Dans le cas où il est souhaité une cellulose oxydée présentant des fonctions acide carboxylique complexées avec des ions calcium (Ca²⁺), plusieurs possibilités existent :
- soit l'oxydation est mise en oeuvre à l'aide de réactifs se présentant sous forme de sels de calcium, notamment hypochlorite de calcium (CaCl₂O₂) et bromure de calcium (Br₂Ca). Dans ce cas, à l'issue de la réaction, les fonctions acide carboxylique se présentent sous la forme COOCa.
- alternativement, il peut être envisagé de réaliser l'incubation en présence de la source de calcium (notamment acétate de calcium ou CaCl₂), après l'oxydation ;
- une troisième possibilité consiste à incuber la compresse dans un milieu contenant une source de calcium, après l'étape de protonation. Là encore, des sources de calcium particulièrement adaptées sont l'acétate de calcium ou le CaCl₂. Dans cette nouvelle étape, les ions Ca²⁺ vont remplacer les protons.

A l'issue de ces étapes d'oxydation, éventuellement de protonation, et éventuellement de complexation des fonctions acide carboxylique avec des ions calcium, les compresses sont lavées et séchées.

Le lavage de la compresse se fait de manière avantageuse dans un milieu contenant un solvant et/ou de l'eau.

Un alcool et/ou de l'eau déminéralisée constituent un milieu privilégié de lavage. Un alcool de type isopropanol permet un séchage ultérieur plus rapide à l'air.

Ces lavages peuvent être répétés et durent généralement quelques heures, avantageusement de 1 à 10 heures.

Dans une ultime étape du procédé selon l'invention, les compresses de cellulose oxydées prêtes à l'emploi sont séchées par tout moyen adapté, notamment à l'air ou sous hotte.

Elles peuvent également subir des traitements liés à l'application visée : conditionnement en emballages individuels, stérilisation, ...

Le conditionnement peut être réalisé sous atmosphère modifié, par exemple avec un taux d'humidité résiduel très faible, ou un gaz neutre pour améliorer la stabilité dans le temps de la compresse.

Les compresses traitées à l'aide du procédé selon l'invention sont avantageusement réalisées à l'aide de fils de viscose tricotés en maille crochet.

Il a été démontré que des compresses à base de cellulose soumises au procédé tel que décrit dans la présente invention présentaient une oxydation homogène supérieure ou égale à 10%, voire 12%, et même à 14%, tout en conservant la structure et la résistance mécanique du textile.

En outre et de manière remarquable par rapport à l'art antérieur, le procédé selon l'invention confère une grande sélectivité vis-à-vis de l'oxydation du groupe alcool primaire porté par le carbone 6 des unités anhydroglucose de la cellulose traitée en acide carboxylique. Ainsi, les compresses obtenues à l'aide du procédé de l'invention présentent une sélectivité d'oxydation de l'alcool primaire supérieure ou égale à 70%, avantageusement à 75%, voire à 80%, à 85%, à 90% voire supérieure ou égale à 95%. Cette grandeur est quantifiable notamment par RMN.

### EXEMPLES DE REALISATION

La présente invention va être illustrée plus avant à l'aide des exemples de réalisation présentés ci-dessous, à l'appui des figures annexées. Toutefois, ces exemples de réalisation ne sont en aucun cas limitatifs.
La figure 1 montre l'évolution du pH du milieu réactionnel au cours de la réaction d'oxydation pour l'essai 1.
La figure 2 montre l'évolution du pH du milieu réactionnel au cours de la réaction d'oxydation pour l'essai 2.
La figure 3 montre l'évolution du pH du milieu réactionnel au cours de la réaction d'oxydation pour l'essai 3.
La figure 4 montre l'évolution du pH du milieu réactionnel au cours de la réaction d'oxydation pour l'essai 4.
La figure 5 montre l'évolution du pH du milieu réactionnel au cours de la réaction d'oxydation pour l'essai 5.
La figure 6 montre l'évolution du pH du milieu réactionnel au cours de la réaction d'oxydation pour l'essai 6.
La figure 7 montre l'évolution du pH du milieu réactionnel au cours de la réaction d'oxydation pour l'essai 7.
La figure 8 montre l'évolution du pH du milieu réactionnel au cours de la réaction d'oxydation pour l'essai 8.
La figure 9 montre l'évolution du pH du milieu réactionnel au cours de la réaction d'oxydation pour l'essai 9.

### 1/ Compresses à base de cellulose :

Les expériences ont été réalisées sur des compresses fabriquées à partir de fils de viscose tricotés avec deux types de maille :
- maille jersey, tricotée avec du fil de 220 dtex à 42 filaments (essai 3) ;
- maille crochet, tricotée avec du fil de 110 dtex à 40 filaments (essais 1 et 2).

Ces tricots présentent un grammage de l'ordre de 67,5g/m².

Pour ces produits, l'étape d'ensimage a lieu au moment de la fabrication du fil, juste avant le bobinage. De fait, tous ces tricots sont donc ensimés.

### 2/ Réaction d'oxydation :

Pour les essais 1 à 4, la compresse de cellulose est placée dans un milieu réactionnel comprenant :
- du bromure de sodium (NaBr) ;
- de l'hypochlorite de sodium à 15% de chlore actif (NaOCl) ou javel ;
- du Tempo ou 2,2,6,6-tétraméthylpipéridine-1-oxyde ;
- de l'eau déminéralisée à 4°C.

Pour ces mêmes essais, les réactifs suivants ont été ajoutés dans le milieu réactionnel aux quantités indiquées dans le tableau suivant :

| Réactifs | Nb de moles par mole d'unités anhydroglucose dans l'échantillon de cellulose traité (équivalents) | mg/ml du milieu réactionnel |
|---|---|---|
| Tempo | 0,05 | 1,2 |
| NaBr | 0,787 | 12,5 |
| NaOCl total (ajouté tout au long de la réaction) | 6,97 | 96,75 |

Le milieu réactionnel est composé de 40 ml d'eau déminéralisée pour 1 g de cellulose.

Au début de la réaction, le Tempo et le NaBr sont ajoutés intégralement dans le milieu réactionnel. En revanche, seule une partie du NaOCl (0,486 mole par mole d'unités anhydroglucose dans l'échantillon de cellulose traité (équivalents)) est ajoutée, de manière à obtenir un pH initial compris entre 11 et 12.

La réaction se déroule à 4°C. L'évolution du pH du milieu réactionnel est suivie, et du NaOCl est ajouté dans le milieu réactionnel dans les conditions mentionnées dans le tableau ci-dessous :

| | Essai 1 | Essai 2 | Essai 3 | Essai 4 |
|---|---|---|---|---|
| Cellulose | Crochet | Crochet | Jersey | Jersey |
| Température | 4°C | 4°C | 4°C | Ambiante |
| pH d'ajout du NaOCl | 8,2 | 8,2 | 7,5 | 8,2 |
| Nombre d'ajouts | 8 | 8 | 8 | 8 |
| Nombre de moles par mole d'unités anhydroglucose dans l'échantillon de cellulose traité (équivalents) par ajout | 0,81 | 0,81 | 0,81 | 0,81 |
| Durée d'oxydation | 1392 min | 1412 min | 1404 min | 1383 min |
| pH du palier | 6,36 | 7,51 | 7,3 | 5,11 |

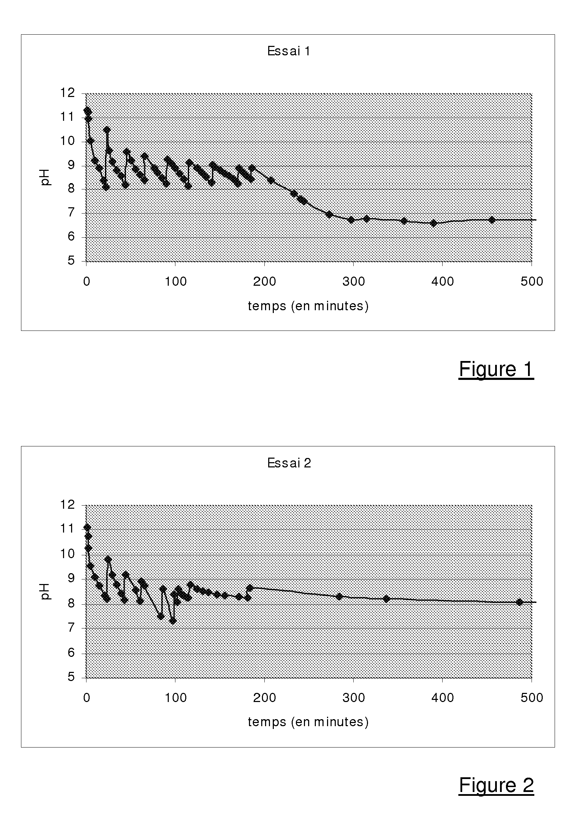

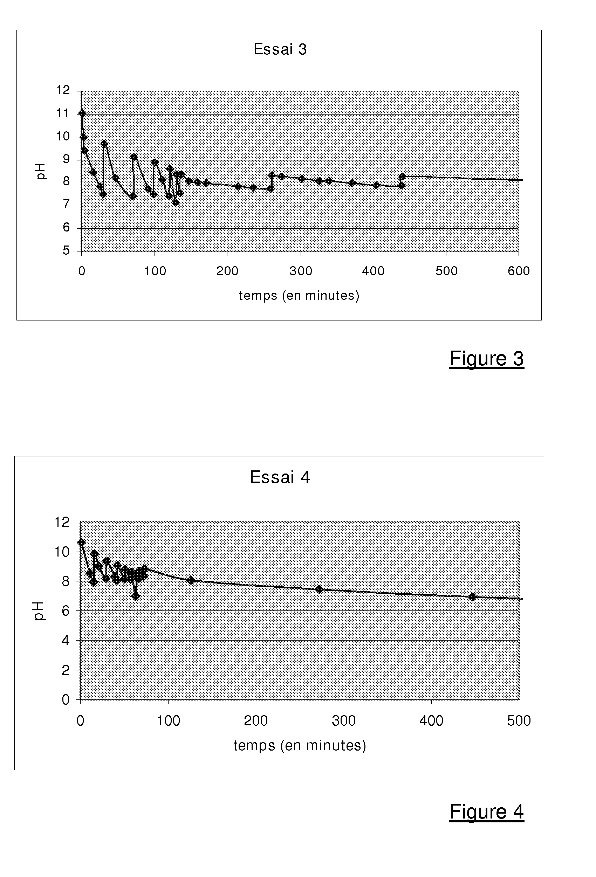

Les variations du pH au cours de la réaction sont représentées aux Figures 1 à 4. Les « remontées » de pH correspondent à l'ajout de NaOCl. La quantité totale de NaOCl a été consommée au cours de 8 ajouts s'étendant sur une période de 3 à 4 heures. Ensuite, la réaction se continue jusqu'à une durée totale de la réaction d'environ 24 heures. A la fin de la réaction, le milieu réactionnel atteint un pH palier compris entre 5 et 8.

### 3/ Réaction de protonation :

La réaction d'oxydation est considérée comme étant terminée lorsque le milieu réactionnel passe de jaune à blanc. Elle a été stoppée par ajout de méthanol.

Le milieu réactionnel oxydant a été vidangé et remplacé par un milieu de protonation dans les conditions suivantes :

| HCl 1 mol/l (N) | Essai 1 | Essai 2 | Essai 3 | Essai 4 |
|---|---|---|---|---|
| Nb de bains | 2 | 2 | 2 | 2 |
| Durée totale | 7h | 3h | 3h | 4h50 |

### 4/ Lavages des compresses:

Les compresses ont subi des lavages dans les conditions suivantes :

| | Essai 1 | Essai 2 | Essai 3 | Essai 4 |
|---|---|---|---|---|
| 1^{er} lavage | Eau brute pendant 5 minutes | Bain eau déminéralisée / isopropanol (50/50) | Bain eau déminéralisée / isopropanol (50/50) | Bain eau déminéralisée / isopropanol (50/50) |
| | | Durée : 1 h 30 | Durée : 1 h 30 | Durée :1 h 40 |
| 2^{ème} lavage | - | Bain d'isopropanol | Bain d'isopropanol | Bain d'isopropanol |
| | | Durée : 1 h | Durée : 1 h | Durée : 1 h |

Les compresses ont alors été séchées à l'air libre sous hotte aspirante.

### 5/ Résultats :

Les compresses de cellulose oxydée ont été testées pour différentes propriétés recherchées :

| | Essai 1 | Essai 2 | Essai 3 | Essai 4 | Référence |
|---|---|---|---|---|---|
| Taux d'oxydation | 8,50 % | 17,70 % | 17,30 % | 14,70 % | Entre 14 et 25% |
| Solubilisation totale dans la soude | +/- | + | + | + | + |
| Résistance mécanique | R- | R++ | R++ | R+ | R+ |
| Aspect du textile | Présence de trous | Intègre | Léger démaillage, pas de trous | Léger démaillage | Intègre |

La référence correspond à une compresse de cellulose oxydée à l'aide des procédés de l'art antérieur, à savoir une oxydation à l'aide de NO₂ et en présence de solvant conformément au document EP 0 492 990.

On constate que le lavage à l'eau brute ne donne pas des résultats satisfaisants : la compresse correspondante (essai 1) présente des trous et un taux d'oxydation insuffisant.

Les compresses des essais 2 et 3 donnent des résultats tout à fait satisfaisants, tant en termes de degré d'oxydation que de résistance mécanique, validant ainsi la faisabilité et l'intérêt de la présente invention. Les mailles « crochet » semblent mieux tenir que les mailles «jersey» plus lâches, mais fondamentalement, le choix du textile n'a pas d'influence sur le taux d'oxydation.

En outre et de manière attendue, la comparaison des essais 3 et 4 confirment que des conditions de basse température (4°C versus température ambiante) donnent de meilleurs résultats, tant en termes de taux d'oxydation que de résistance mécanique. Toutefois, les prothèses obtenues dans les conditions de l'essai 4 restent acceptables.

Des tests complémentaires ont été réalisés pour vérifier que des modifications mineures dans les conditions expérimentales, à l'intérieur du champ de protection recherché, restaient acceptables. Ainsi, il a été testé l'effet des variations affectant :
- la quantité d'eau dans le milieu réactionnel (essais 5 et 6 à comparer avec l'essai 3) ;
- la quantité d'eau de javel (essais 5 et 7 à comparer avec l'essai 3) ;
- la quantité du catalyseur Tempo (essai 7 à comparer avec l'essai 3) ;
- l'utilisation de NaCl en lieu et place de NaBr (essai 8 à comparer à l'essai 6) ;
- l'utilisation d'un fil non tricoté 110 dtex, 40 filaments en lieu et place du tricot maille crochet (essai 9 à comparer à l'essai 2) ;
- la complexation avec des ions calcium (essai 10 à comparer à l'essai 2).

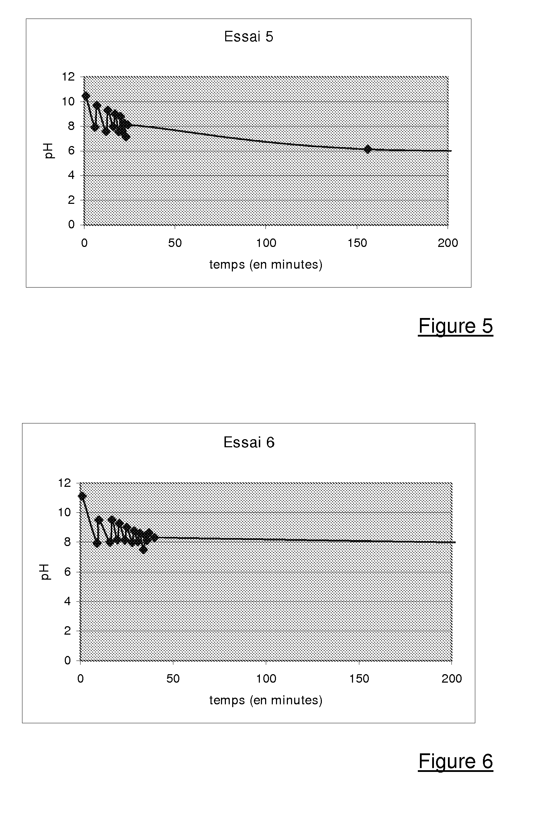

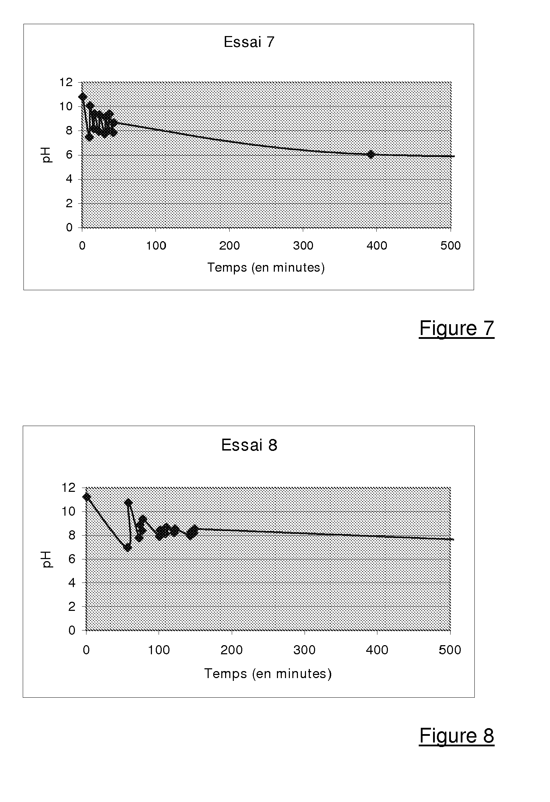

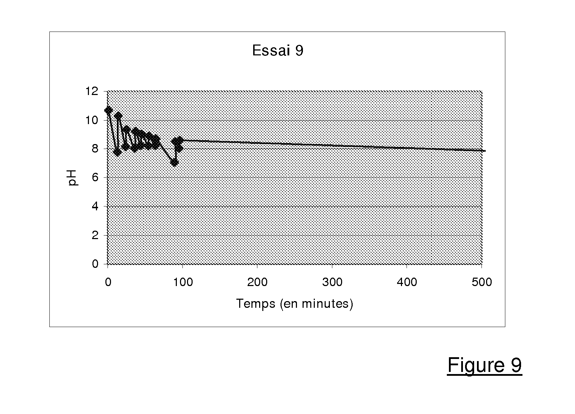

Les résultats obtenus sont présentés dans les tableaux suivants, ainsi que dans les figures 5 à 9. On constate que dans toutes ces conditions modifiées, on obtient des compresses avec des propriétés moins favorables, notamment en termes de taux d'oxydation et de résistance mécanique, que dans les conditions déterminées comme les plus favorables, mais qui restent toutefois acceptables pour l'application visée.

### Essai 5

| Réactifs | Nb de moles par mole d'unités anhydroglucose dans l'échantillon de cellulose traité (équivalents) | mg/ml du milieu réactionnel |
|---|---|---|
| Tempo | 0,05 | 4,8 |
| NaBr | 0,787 | 50 |
| NaOCl total (ajouté tout au long de la réaction) | 5,35 | 297 |

Le milieu réactionnel est composé de 10 ml d'eau déminéralisée pour 1 g de cellulose.

### Essai 6

| Réactifs | Nb de moles par mole d'unités anhydroglucose dans l'échantillon de cellulose traité (équivalents) | mg/ml du milieu réactionnel |
|---|---|---|
| Tempo | 0,05 | 3,2 |
| NaBr | 0,787 | 33,3 |
| NaOCl total (ajouté tout au long de la réaction) | 6,97 | 258 |

Le milieu réactionnel est composé de 15 ml d'eau déminéralisée.

### Essai 7

| Réactifs | Nb de moles par mole d'unités anhydroglucose dans l'échantillon de cellulose traité (équivalents) | mg/ml du milieu réactionnel |
|---|---|---|
| Tempo | 0,06 | 3,85 |
| NaBr | 0,787 | 33,3 |
| NaOCl total (ajouté tout au long de la réaction) | 5,35 | 198 |

Le milieu réactionnel est composé de 15 ml d'eau déminéralisée.

| | Essai 5 | Essai 6 | Essai 7 |
|---|---|---|---|
| Cellulose | Jersey | Jersey | Jersey |
| Température | 4°C | 4°C | 4°C |
| pH d'ajout du NaOCl | 8,2 | 8,2 | 8,2 |
| Nombre d'ajouts | 6 | 8 | 6 |
| Nombre de moles par mole d'unités anhydroglucose dans l'échantillon de cellulose traité (équivalents) par ajout | 0,81 | 0,81 | 0,81 |
| Durée d'oxydation | 1402 min | 1417 min | 1422 min |
| pH du palier | 5,27 | 5,59 | 5,84 |

L'oxydation a été stoppée avec du méthanol dans le cas de l'essai 5 et avec de l'éthanol pour les essais 6 et 7.

### Protonation :

| HCl 1 mol/l (N) | Essai 5 | Essai 6 | Essai 7 |
|---|---|---|---|
| Nb de bains | 2 | 2 | 3 |
| Durée totale | 2h40 | 3 h 55 | 3h25 |

### Lavages :

| | Essai 5 | Essai 6 | Essai 7 |
|---|---|---|---|
| 1^{er} lavage | Bain eau déminéralisée / isopropanol (50/50) | Bain eau déminéralisée / isopropanol (50/50) | Bain eau déminéralisée / isopropanol (50/50) |
| | Durée : 1 h 20 | Durée : 1 h | Durée :1 h 30 |
| 2^{ème} lavage | Bain d'isopropanol | Bain d'isopropanol | Bain d'isopropanol |
| | Durée : 1 h 40 | Durée : 1 h 30 | Durée : 1 h 40 |

Le séchage est effectué sous hotte aspirante.

### Résultats :

| | Essai 5 | Essai 6 | Essai 7 |
|---|---|---|---|
| Taux d'oxydation | 13,60 % | 15,20 % | 14,30 % |
| Solubilisation totale dans la soude | + | + | + |
| Résistance mécanique | R ++ | R ++ | R ++ |
| Aspect du textile | Léger démaillage | Léger démaillage | Léger démaillage |

### Essai 8

| Réactifs | Nb de moles par mole d'unités anhydroglucose dans l'échantillon de cellulose traité (équivalents). | mg/ml du milieu réactionnel |
|---|---|---|
| Tempo | 0,05 | 3,2 |
| NaCl | 1,39 | 33,3 |
| NaOCl total (ajouté tout au long de la réaction) | 6,97 | 258 |

Le milieu réactionnel est composé de 15 ml d'eau déminéralisée pour 1 g de cellulose.

| | Essai 8 |
|---|---|
| Cellulose | Jersey |
| Température | 4°C |
| pH d'ajout du NaOCl | 8,2 |
| Nombre d'ajouts | 8 |
| Nombre de moles par mole d'unités anhydroglucose dans l'échantillon de cellulose traité (équivalents) par ajout | 0,81 |
| Durée d'oxydation | 1412 min |
| pH du palier | 5,27 |

L'oxydation a été stoppée avec de l'éthanol.

### Protonation :

| HCl 1 mol/l (N) | Essai 8 |
|---|---|
| Nb de bains | 2 |
| Durée totale | 2 h 50 |

### Lavages :

| | Essai 8 |
|---|---|
| 1^{er} lavage | Bain eau déminéralisée / isopropanol (50/50) |
| | Durée : 1 h 10 |
| 2^{ème} lavage | Bain d'isopropanol |
| | Durée : 1 h 45 |

Le séchage est effectué sous hotte aspirante.

### Résultats :

| | Essai 8 |
|---|---|
| Taux d'oxydation | 13,80 % |
| Solubilisation totale dans la soude | + |
| Résistance mécanique | R +/- |
| Aspect du textile | Léger démaillage |

### Essai 9 :

| Réactifs | Nb de moles par mole d'unités anhydroglucose dans l'échantillon de cellulose traité (équivalents) | mg/ml du milieu réactionnel |
|---|---|---|
| Tempo | 0,05 | 1,2 |
| NaBr | 0,787 | 12,5 |
| NaOCl total (ajouté tout au long de la réaction) | 6,97 | 96,75 |

Le milieu réactionnel est composé de 40 ml d'eau déminéralisée pour 1 g de cellulose.

| | Essai 9 |
|---|---|
| Cellulose | Fil de 110 dtex, 40 filaments |
| Température | 4°C |
| pH d'ajout du NaOCl | 8,2 |
| Nombre d'ajouts | 8 |
| Nombre de moles par mole d'unités anhydroglucose dans l'échantillon de cellulose traité (équivalents) par ajout | 0,81 |
| Durée d'oxydation | 1406 min |
| pH du palier | 6,13 |

L'oxydation a été stoppée avec de l'éthanol.

### Protonation :

| HCl 1 mol/l (N) | Essai 9 |
|---|---|
| Nb de bains | 2 |
| Durée totale | 3 h 25 |

### Lavages :

| | Essai 9 |
|---|---|
| 1^{er} lavage | Bain eau déminéralisée / isopropanol (50/50) Durée : 1 h 55 |
| 2^{ème} lavage | Bain d'isopropanol Durée : 1 h 25 |

Le séchage est effectué sous hotte aspirante.

### Résultats :

| | Essai 9 |
|---|---|
| Taux d'oxydation | 13,60 % |
| Solubilisation totale dans la soude | + |

### Essai 10 : Compresse complexée à l'aide d'ions calcium.

La compresse a été traitée dans les mêmes conditions que celle de l'essai 2. Cette compresse est immergée dans un bain comprenant 3,33 moles d'acétate de calcium par mole d'unités anhydroglucose dans l'échantillon de cellulose traité (équivalents) dans un mélange eau déminéralisée / isopropanol (50/50), pendant 2 heures.

Deux lavages de 30 min, chacun dans des bains d'isopropanol, sont ensuite effectués.

### Résultats :

| | Essai 10 |
|---|---|
| Taux d'oxydation | 0,40 % |
| Solubilisation totale dans la soude | - |
| Résistance mécanique | R + |
| Aspect du textile | Intègre |

La chute du taux d'oxydation (0,4% contre 17,7%) montre que les protons ont été remplacés par des ions calcium.

Plus précisément, des dosages de calcium ont été réalisés pour valider l'échange des fonctions acide par le calcium : Ainsi, le taux de calcium sur cet échantillon a été estimé à 6,6%, ce qui correspond à un taux de substitution des fonctions acide par le calcium de plus de 90%.

Par ailleurs, la compresse ainsi obtenue présente toutes les propriétés recherchées.

## Revendications

1. Procédé de préparation d'une compresse de cellulose oxydée comprenant l'oxydation de la compresse à base de cellulose à l'aide d'un hypohalite, en présence d'un sel d'oxoammonium.

2. Procédé de préparation d'une compresse selon la revendication 1 ***caractérisé*** en ce que l'oxydation est réalisée dans un milieu réactionnel dont le pH initial est compris entre 10 et 12.

3. Procédé de préparation d'une compresse selon la revendication 2 ***caractérisé*** en ce que le milieu réactionnel est enrichi en hypohalite lorsque son pH atteint une valeur comprise entre 7 et 9.

4. Procédé de préparation d'une compresse selon la revendication 3 ***caractérisé*** en ce que l'addition d'hypohalite dans le milieu est stoppée lorsqu'une quantité totale en hypohalite comprise entre 5 et 7 moles par mole d'unités anhydroglucose dans la cellulose (équivalents) a été ajoutée au milieu.

5. Procédé de préparation d'une compresse selon l'une des revendications précédentes ***caractérisé*** en ce que le sel d'oxoammonium est du 2,2,6,6-tétraméthylpipéridine-1-oxyde (Tempo).

6. Procédé de préparation d'une compresse selon l'une des revendications précédentes ***caractérisé*** en ce que l'hypohalite est de l'hypochlorite de sodium.

7. Procédé de préparation d'une compresse selon l'une des revendications précédentes ***caractérisé*** en ce que l'oxydation est réalisée en présence d'un sel régénérateur de l'hypohalite.

8. Procédé de préparation d'une compresse selon l'une des revendications précédentes ***caractérisé*** en ce que l'oxydation est réalisée en présence d'une source d'ions calcium.

9. Procédé de préparation d'une compresse selon l'une des revendications précédentes ***caractérisé*** en ce que l'oxydation est stoppée lorsque le milieu réactionnel atteint un pH palier compris entre 5 et 8.

10. Procédé de préparation d'une compresse selon l'une des revendications précédentes ***caractérisé*** en ce que l'oxydation est stoppée par ajout d'un excès d'alcool primaire.

11. Procédé de préparation d'une compresse selon l'une des revendications précédentes ***caractérisé*** en ce que l'oxydation est réalisée à une température égale à 4°C.

12. Procédé de préparation d'une compresse selon l'une des revendications précédentes ***caractérisé*** en ce que, après arrêt de l'oxydation, le milieu réactionnel est éliminé par vidange.

13. Procédé de préparation d'une compresse selon l'une des revendications précédentes ***caractérisé*** en ce que, après oxydation, la compresse est incubée dans un milieu de protonation, pendant 1 à 10 heures.

14. Procédé de préparation d'une compresse selon l'une des revendications précédentes ***caractérisé*** en ce que, après oxydation ou après protonation, la compresse est incubée dans un milieu riche en ions calcium.

15. Procédé de préparation d'une compresse selon l'une des revendications précédentes ***caractérisé*** en ce que, avant son oxydation, la compresse à base de cellulose est soumise à une étape de désensimage.

16. Procédé de préparation d'une compresse selon l'une des revendications précédentes ***caractérisé*** en ce qu'à l'issue des étapes, la compresse à base de cellulose est lavée et séchée.

17. Compresse hémostatique et résorbable obtenue à l'aide du procédé selon l'une des revendications 1 à 16 ***caractérisée*** en ce qu'elle présente un taux d'oxydation supérieur ou égal à 10% et une sélectivité d'oxydation de l'alcool primaire supérieure ou égale à 70%.

18. Compresse hémostatique et résorbable selon la revendication 17 ***caractérisée*** en ce qu'elle est obtenue à partir d'un textile de fils de viscose.

## Patentansprüche

1. Verfahren zur Herstellung einer Kompresse aus oxidiertem Zellstoff, die Oxidation der Kompresse auf Zellstoffbasis mittels eines Hypohalits im Beisein eines Oxoammoniumsalzes umfassend.

2. Verfahren zur Herstellung einer Kompresse nach Anspruch 1, **dadurch gekennzeichnet, dass** die Oxidation in einem Reaktionsmedium erfolgt, dessen Anfangs-pH zwischen 10 und 12 beträgt.

3. Verfahren zur Herstellung einer Kompresse nach Anspruch 2, **dadurch gekennzeichnet, dass** das Reaktionsmedium mit Hypohalit angereichert wird, wenn sein pH einen Wert zwischen 7 und 9 erreicht.

4. Verfahren zur Herstellung einer Kompresse nach Anspruch 3, **dadurch gekennzeichnet, dass** der Zusatz von Hypohalit in das Medium gestoppt wird, wenn eine Gesamtmenge an Hypohalit zwischen 5 und 7 Mol pro Mol von Anhydroglukose-Einheiten im Zellstoff (Äquivalenten) dem Medium zugesetzt wurde.

5. Verfahren zur Herstellung einer Kompresse nach einem der vorhergehenden Ansprüche, **dadurch gekennzeichnet, dass** das Oxoammoniumsalz 2,2,6,6-Tetramethylpiperidin-1-oxid (Tempo) ist.

6. Verfahren zur Herstellung einer Kompresse nach einem der vorhergehenden Ansprüche, **dadurch gekennzeichnet, dass** das Hypohalit Natriumhypohalit ist.

7. Verfahren zur Herstellung einer Kompresse nach einem der vorhergehenden Ansprüche, **dadurch gekennzeichnet, dass** die Oxidation im Beisein eines Hypohalit-Rengeneratorsalzes erfolgt.

8. Verfahren zur Herstellung einer Kompresse nach einem der vorhergehenden Ansprüche, **dadurch gekennzeichnet, dass** die Oxidation im Beisein einer Kalziumionenquelle erfolgt.

9. Verfahren zur Herstellung einer Kompresse nach einem der vorhergehenden Ansprüche, **dadurch gekennzeichnet, dass** die Oxidation gestoppt wird, wenn das Reaktionsmedium ein pH-Stadium zwischen 5 und 8 erreicht.

10. Verfahren zur Herstellung einer Kompresse nach einem der vorhergehenden Ansprüche, **dadurch gekennzeichnet, dass** die Oxidation durch Zusatz eines Überschusses an primärem Alkohol gestoppt wird.

11. Verfahren zur Herstellung einer Kompresse nach einem der vorhergehenden Ansprüche, **dadurch gekennzeichnet, dass** die Oxidation bei einer Temperatur von gleich 4°C erfolgt.

12. Verfahren zur Herstellung einer Kompresse nach einem der vorhergehenden Ansprüche, **dadurch gekennzeichnet, dass** nach dem Anhalten der Oxidation das Reaktionsmedium durch Entleerung entfernt wird.

13. Verfahren zur Herstellung einer Kompresse nach einem der vorhergehenden Ansprüche, **dadurch gekennzeichnet, dass** nach der Oxidation die Kompresse 1 bis 10 Stunden lang in einem Protonierungsmedium inkubiert wird.

14. Verfahren zur Herstellung einer Kompresse nach einem der vorhergehenden Ansprüche, **dadurch gekennzeichnet, dass** nach der Oxidation oder nach der Protonierung die Kompresse in einem an Kalziumionen reichen Medium inkubiert wird.

15. Verfahren zur Herstellung einer Kompresse nach einem der vorhergehenden Ansprüche, **dadurch gekennzeichnet, dass** die Kompresse auf Zellstoffbasis vor ihrer Oxidation einem Entschmälzungsschritt unterzogen wird.

16. Verfahren zur Herstellung einer Kompresse nach einem der vorhergehenden Ansprüche, **dadurch gekennzeichnet, dass** die Kompresse auf Zellstoffbasis am Ende der Schritte gewaschen und getrocknet wird.

17. Hämostatische und resorbierbare Kompresse, die mittels des Verfahrens nach einem der Ansprüche 1 bis 16 erhalten ist, **dadurch gekennzeichnet, dass** sie einen Oxidationsgrad von über oder gleich 10% und eine Oxidationsselektivität für den primären Alkohol von über oder gleich 70% aufweist.

18. Hämostatische und resorbierbare Kompresse nach Anspruch 17, **dadurch gekennzeichnet, dass** sie aus einem Gewebe aus Viskosefäden gewonnen ist.

## Claims

1. Method for preparing an oxidized cellulose compress comprising the oxidation of the cellulose based compress with a hypohalite, in the presence of an oxoammonium salt.

2. Method for preparing a compress according to Claim 1, ***characterized*** in that the oxidation is carried out in a reaction medium of which the initial pH is between 10 and 12.

3. Method for preparing a compress according to Claim 2, ***characterized*** in that the reaction medium is enriched with hypohalite when its pH reaches a value of between 7 and 9.

4. Method for preparing a compress according to Claim 3, ***characterized*** in that the addition of hypohalite to the medium is stopped when a total quantity of hypohalite between 5 and 7 moles per mole of anhydro-glucose units in the cellulose (equivalents) has been added to the medium.

5. Method for preparing a compress according to one of the preceding claims, ***characterized*** in that the oxoammonium salt is 2,2,6,6-tetramethylpiperidine-1-oxide (Tempo).

6. Method for preparing a compress according to one of the preceding claims, ***characterized*** in that the hypohalite is sodium hypochlorite.

7. Method for preparing a compress according to one of the preceding claims, ***characterized*** in that the oxidation is carried out in the presence of a hypohalite regenerating salt.

8. Method for preparing a compress according to one of the preceding claims, ***characterized*** in that the oxidation is carried out in the presence of a source of calcium ions.

9. Method for preparing a compress according to one of the preceding claims, ***characterized*** in that the oxidation is stopped when the reaction medium reaches a pH plateau between 5 and 8.

10. Method for preparing a compress according to one of the preceding claims, ***characterized*** in that the oxidation is stopped by the addition of an excess of primary alcohol.

11. Method for preparing a compress according to one of the preceding claims, ***characterized*** in that the oxidation is carried out at a temperature of 4°C.

12. Method for preparing a compress according to one of the preceding claims, ***characterized*** in that, after the oxidation is stopped, the reaction medium is removed by drainage.

13. Method for preparing a compress according to one of the preceding claims, ***characterized*** in that, after oxidation, the compress is incubated in a protonation medium for 1 to 10 hours.

14. Method for preparing a compress according to one of the preceding claims, ***characterized*** in that, after oxidation or after protonation, the compress is incubated in a medium rich in calcium ions.

15. Method for preparing a compress according to one of the preceding claims, ***characterized*** in that, after its oxidation, the cellulose based compress is subjected to a de-oiling step.

16. Method for preparing a compress according to one of the preceding claims, ***characterized*** in that on completion of the steps, the cellulose based compress is washed and dried.

17. Haemostatic and resorbable compress obtained by the method according to one of Claims 1 to 16, **characterized in that** it has an oxidation rate of 10% or higher and a primary alcohol oxidation selectivity of 70% or higher.

18. Haemostatic and resorbable compress according to Claim 17 ***characterized*** in that it is obtained from a viscose yarn textile.
